**Europäisches Patentamt**

**European Patent Office**

**Office européen des brevets**

(11) Veröffentlichungsnummer: **0 017 712**
**B1**

(12) EUROPÄISCHE PATENTSCHRIFT

(45) Veröffentlichungstag der Patentschrift: **28.08.85**

(21) Anmeldenummer: **80100598.4**

(22) Anmeldetag: **06.02.80**

(51) Int. Cl.⁴: **G 01 N 15/04, C 02 F 3/00, G 01 N 33/18**

(54) Verfahren und Vorrichtung zur Messung des Feststoffgehaltes einer Flüssigkeit.

(30) Priorität: **31.03.79 DE 2913058**

(43) Veröffentlichungstag der Anmeldung:
**29.10.80 Patentblatt 80/22**

(45) Bekanntmachung des Hinweises auf die Patenterteilung:
**28.08.85 Patentblatt 85/35**

(84) Benannte Vertragsstaaten:
**AT BE CH DE FR GB IT NL SE**

(56) Entgegenhaltungen:
**DE-A-1 498 715**
**FR-A-2 114 296**
**GB-A-1 451 661**
**US-A-3 714 445**
**US-A-3 953 136**

(73) Patentinhaber: **EPCO Gesellschaft mit beschränkter Haftung für Handel und Verwaltung**
**Am alten Schacht 8**
**D-4100 Duisburg 17 (DE)**

(72) Erfinder: **Schniewind, Manfred**
**Emdenstrasse 21**
**D-4300 Essen 1 (DE)**

(74) Vertreter: **Bonsmann, Manfred, Dipl.-Ing.**
**Kaldenkirchener Strasse 35a**
**D-4050 Mönchengladbach 1 (DE)**

**Beschreibung**

Die Erfindung betrifft eine Vorrichtung zum automatischen Messen des absetzbaren Feststoffgehaltes eines Abwasser-Belebtschlamm-Gemisches, mit einem lichtdurchlässigen, senkrechten Meßrohr, das durch eine Pumpe mit der Flüssigkeit gefüllt wird, und mit einem fotoelektrischen Abtastkopf zum Auffinden der Lage der Grenzschicht der abgelagerten Feststoffschicht, sowie ein Verfahren zum automatischen Messen des absetzbaren Feststoffgehaltes eines Abwasser-Belebtschlamm-Gemisches mit einer solchen Vorrichtung.

Eine derartige Vorrichtung ist aus der DE—A— 14 98 715 bekannt. Diese bekannte Vorrichtung ist konstruktiv aufwendig, da für eine einwandfreie Funktion mindestens zwei Ventile erforderlich sind und ferner eine Reinigungsvorrichtung, die in das Meßrohr eingefahren werden muß, um die Innenwandung des Meßrohres sauber zu halten. Die Ventile und die Reinigungsvorrichtung erfordern einen zusätzlichen Steueraufwand. Ferner wird bei dieser bekannten Vorrichtung die Feststoffe enthaltende Flüssigkeit in das Meßrohr durch eine Pumpe gefördert, die die Feststoffe in der Flüssigkeit durchmischt und hierdurch verändert. Auch können Pumpe und Ventile durch die in der Flüssigkeit enthaltenen Feststoffe leicht verschmutzen.

Auf dem weiteren Gebiet der fotoelektrischen Messung von Flüssigkeiten ist durch die US—A—37 14 445 eine zu einer diskontinuierlichen Messung geeignete Meßvorrichtung bekannt geworden, bei der eine Flüssigkeitsprobe in ein Meßrohr gepumpt und nach der Messung durch einen Kolben zurückbefördert wird, wobei das Meßrohr gleichzeitig durch den Kolben gereinigt wird. Die bekannte Vorrichtung ist jedoch nicht für das Messen einer Grenzschicht in einem Abwasser-Belebtschlamm-Gemisch vorgesehen, sondern es wird zur Überwachung des Wachsens von Mikrokulturen die optische Dichte gemessen.

Aufgabe der Erfindung ist es, eine Vorrichtung der eingangs genannten Art zu schaffen, bei der der konstruktive Aufwand verringert ist, ein geringer Steuer- und/oder Regelaufwand entsteht und sich eine zusätzliche Reinigungsvorrichtung, wie sie bei der bekannten Vorrichtung entsprechend der Gattung des Oberbegriffes des Anspruches 1 erforderlich ist, erübrigt. Darüber hinaus ist es Aufgabe der Erfindung, die Störanfälligkeit und den Wartungsaufwand einer Vorrichtung der eingangs genannten Art zu vermindern.

Erfindungsgemäß ist vorgesehen, daß im Meßrohr ein Kolben angeordnet ist, durch den die im Meßrohr befindliche Flüssigkeit nach oben hin verdrängbar ist, wodurch die Grenzschicht an dem feststehenden Abtastkopf bewegbar ist.

Bei der Vorrichtung gemäß der Erfindung wird ein Transportmechanismus für die fotoelektrische Vorrichtung dadurch überflüssig gemacht, daß die Grenzschicht durch den Kolben an diese herangeführt wird. Gleichzeitig erübrigen sich eine zusätzliche Reinigungsvorrichtung und Ventile. Die Vorrichtung weist eine geringe Störanfälligkeit auf und kommt mit einem geringen Steuer- und Regelaufwand aus. Das Meßrohr kann übliche standardisierte Größen aufweisen, und ein weiterer unterer Verschluß des Meßrohres ist nicht erforderlich, da der Kolben den unteren Verschluß bildet.

Vorteilhafte Ausgestaltungen der Erfindung sind in den Unteransprüchen aufgeführt.

Zwei Ausführungsbeispiele der Erfindung sind in den Zeichnungen schematisch dargestellt und werden im folgenden näher beschrieben. Es zeigen:

Fig. 1 einen senkrechten Schnitt durch ein erstes Ausführungsbeispiel;

Fig. 2 einen vergrößerten senkrechten Schnitt durch den Kolben nach Fig. 1 einschließlich eines Abschnittes des Meßrohres;

Fig. 3 einen waagerechten Schnitt durch Kolben und Meßrohr nach Fig. 1 und 2;

Fig. 4 einen senkrechten Schnitt durch ein zweites Ausführungsbeispiel;

Fig. 5 bis 9 vergrößerte Ausschnitte aus Fig. 4 in verschiedenen Arbeitsstellungen und

Fig. 10 einen Stopfen zur Trennung der Feststoffschicht von der darüber befindlichen Flüssigkeit.

Eine Flüssigkeitszu- und -abflußleitung 2, die vertikal angeordnet ist oder zumindest ein Gefälle aufweist, reicht mit ihrer unteren Öffnung in eine Flüssigkeit F an einer Stelle hinein, die als Probenahmestelle dient und eine Rinne zwischen zwei Becken einer Abwasserreinigungsanlage sein kann. Die Leitung 2 führt zu der Unterseite bzw. zum Boden eines Überlaufgefäßes 8, in dessen Boden ferner ein Meßrohr 4 kalibriertes Präzisionsrohr aus Glas mit Graduierung mündet, dessen Längsachse vertikal angeordnet ist. Das Meßrohr 4 ragt mit seiner Wandung einige Zentimeter in das Gefäß 8 hinein, und ist an seiner Unterseite durch einen Kolben 10 verschlossen, der durch eine nicht gezeigte Vorrichtung pneumatisch, hydraulisch oder durch ein Gestänge im Meßrohr auf- und abfahrbar ist. Höhe H und Breite B des Meßrohrs 4 entsprechen einem standardisierten Rohr bzw. Küvette zur Messung des Feststoffgehaltes einer Flüssigkeit.

Von der Oberseite des Überlaufgefäßes 8 geht eine Leitung 13 azs, die zu einer Pumpe 1 führt, die die im Überlaufgefäß befindliche Luft absaugt und durch eine Regeleinrichtung 14 gesteuert wird, die auch weitere Teile der Vorrichtung steuert. Die Pumpe 1 kommt mit Abwasser nicht in Berührung, so daß sie nicht verschmutzen und schnell verschleißen kann. In das Überlaufgefäß 8 reicht von oben eine Schalteinrichtung 3 hinein, die eine Elektrode aufweist und, sobald der im Überlaufgefäß befindliche Flüssigkeitsspiegel die Elektrode erreicht, einen Impuls an die Regeleinrichtung 14 gibt, wonach die Pumpe 1 abgeschaltet wird.

Ein das Meßrohr 4 außen umgebender Abtastkopf 7 bildet eine Lichtschranke durch eine Lichtquelle 11 und eine Fotozelle 15. Der Abtastkopf 7 ist längs des Meßrohrs auf- und abfahrbar und durch die angeschlossene Regeleinrichtung 14 so geschaltet, daß der Abtastkopf 7 stehen bleibt, sobald die Lichtschranke durch eine im Maßrohr 4 angelagerte Feststoffschicht 9 (z.B. Belebtschlamm) unterbrochen wird. Dadurch bleibt der Abtastkopf 7 in Höhe der Oberseite 6 (Grenzschicht) der Feststoffschicht 9 stehen. Der Abtastkopf 7 wird durch eine nicht dargestellte Höhenverstellung (z.B. Motor über eine nicht gezeigte Spindel) bewegt, und durch einen Präzisionspotentiometer an der Abtriebswelle wird die genaue Stellung des Abtastkopfes 7 angezeigt. Die Stellung des Abtastkopfes 7 und damit die Höhe der Feststoffschicht 9 wird auf ein Anzeigegerät 16 übertragen, und ferner werden diese Daten in einen Speicher 17 eingebracht. Es kann durch die Vorrichtung nicht nur die abgelagerte Feststoffschicht nach einer bestimmten Absetzzeit automatisch gemessen werden, und dies quasi kontinuierlich in kurzen Zeitabständen, sondern es kann auch durch das Anzeigegerät 16 die Absatzgeschwindigkeit dargestellt werden.

Der Kolben 10 weist an seinem Umfang koaxiale ringförmige Elemente 12 auf, die aus einem elastischen Material, insbesondere aus Gummi oder Kunststoff, bestehen und an der Innenwandung des Meßrohrs 4 abdichtend anliegen. Diese am Kolben befestigten Elemente sind derart aufgebaut, daß sie während der Bewegung des Kolbens 10 die Meßrohrwandung reinigen.

Die Vorrichtung wird durch die Regeleinrichtung 14 wie folgt gesteuert:

Durch Einschalten der Pumpe 1 wird aus dem Überlaufgefäß 8 Luft abgesaugt und dadurch Flüssigkeit von der Probenahmestelle durch die Leitung 2 in das Gefäß 8 eingesaugt. Sobald der Flüssigkeitsspiegel im Gefäß 8 den oberen Rand des Meßrohrs 4 überschritten hat, füllt sich das Meßrohr, und danach steigt der Flüssigkeitsspiegel weiter an bis er die Elektrode der Schalteinrichtung 3 erreicht hat. Das durch den Flüssigkeitsspiegel erzeugte Signal wird von der Schalteinrichtung 3 zur Regeleinrichtung 14 gegeben und bewirkt ein Abschalten der Pumpe 1. Daraufhin strömt die im Gefäß 8 befindliche Flüssigkeit durch die Leitung 2 aufgrund ihres Gewichtes zur Probenahmestelle zurück, wobei durch die stillstehende Pumpe 1 Luft nachströmt. Falls die Bauart der Pumpe 1 ein Durchströmen von Luft während des Stillstandes nicht zuläßt, oder falls das Zurückfließen der im Gefäß 8 befindlichen Flüssigkeit beschleunigt werden soll, kann auch die Pumpe derart eingeschaltet werden, daß sie Luft in das Gefäß 8 hineindrückt. Die im Meßrohr 4 befindliche Flüssigkeit 5 (z.B. Abwasser) verbleibt dort eine bestimmte Zeit, damit die in der Flüssigkeit befindlichen Feststoffe sich absetzen können. Während der Absetzzeit kann der Abtastkopf 7 der sich nach unten verlagernden Oberseite 6 der Feststoffschicht 9 folgen und dem Abzeigegerät 16 Werte übermitteln, die zu einer Aufzeichnung eines Weg-Zeit-Diagramms führen. Alternativ kann auch der Abtastkopf 7 eine bestimmte Zeit lang stehen bleiben und erst danach die Oberseite der Feststoffschicht 9 aufsuchen, wonach die durch den Abtastkopf zurückgelegte Strecke einem Anzeigegerät übermittelt wird.

Nach dem Meßvorgang durch den Abtastkopf 7 wird der Kolben 10 aus seiner bisherigen unteren Stellung nach oben bis zum oberem Rand des Meßrohres 4 gefahren, wodurch die im Meßrohr 4 befindliche Flüssigkeit in das Überlaufgefäß 8 gedrückt wird und von diesem durch die Leitung 2 abläuft. Durch das Hochfahren des Kolbens 10 wird zusätzlich die Innenwandung des Meßrohrs gereinigt. Danach fährt der Kolben 10 wieder in seine untere Stellung, und die Vorrichtung ist für einen darauffolgenden Arbeitszyklus bereit.

Vor einem neuen Meßzyklus erzeugt die Pumpe 1 einen Überdruck. Dadurch wird das während der Meßzeit in das Ansaugrohr eingedrungene Feststoff-Flüssigkeitsgemisch herausgedrückt. Hierdurch wird gewährleistet, daß für jeden Meßzyklus frisches Meßgut in das Meßsystem gelangt.

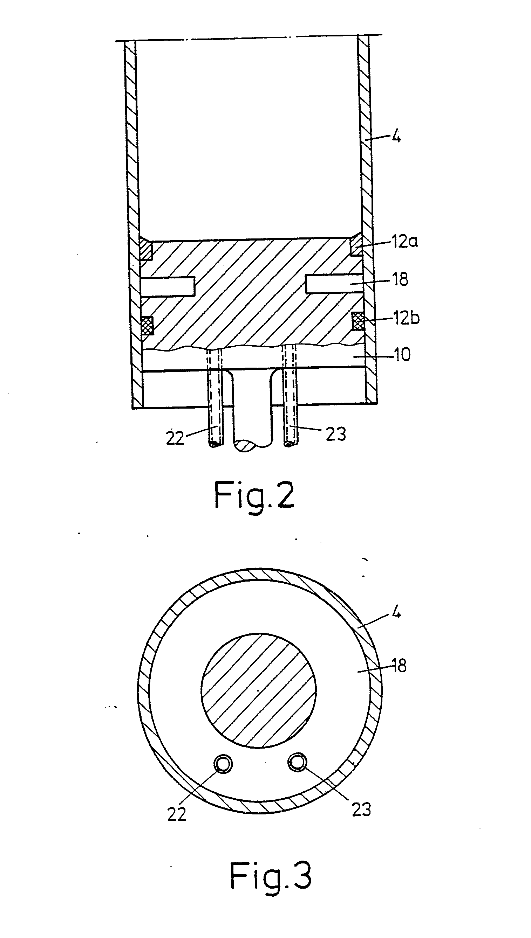

Der in Fig. 2 und 3 gezeigte Kolben besitzt neben dem Abstreifring 12a und dem Dichtring 12b eine Nut 18 zur Aufnahme einer Reinigungsund/oder Gleitlösung.

Die Reinigungsnut kann ausschließlich mit Lösung gefüllt sein oder zusätzlich mit schwammartigem Material. Die Lösung kann manuell (Wartungsintervalle) oder auch automatisch mit Hilfe einer kleinen intermediär arbeitenden Schlauchpumpe über Leitungen 22, 23 erneuert werden.

Auf der Zu- und Abflußleitung 2 ist in einem strahlen- oder schalldurchlässigen Bereich außen eine in einem Gehäuse 19 befindliche Strahlenoder Ultraschallquelle 20 aufgesetzt, deren Schallwellen oder Strahlen die Flüssigkeit durchdringen. Je nach dem Anteil des Feststoffes in der Flüssigkeit gelangen mehr oder weniger Strahlen oder Schallwellen zu einem auf der gegenüberliegenden Seite der Leitung 2 im Gehäuse 19 befindlichen Empfänger 21, so daß die Dichte des Feststoffes errechnet werden kann und das Gewicht des Feststoffes bestimmbar ist.

An der Vorrichtung ist ein nicht gezeigter Rechner angeschlossen, dem die Bewegung des Abtastkopfes 7 über einen festgelegten Zeitabschnitt und damit das Absetzverhalten des Feststoffes eingegeben werden. Ferner erhält dieser Rechner die Stellung bzw. Höhe der Abtastkopfes bzw. der Oberseite 6 der Feststoffschicht (Feststoffvolumen) und die Dichte des Feststoffes (Gewicht) in der Zuflußleitung 2 eingegeben. Aus diesen Werten errechnet der Rechner einen einzigen Wert oder mehrere Werte, die über die Nitrifikation, die Denitrifikation und den Sauerstoffbedarf des Abwassers Aufschluß geben.

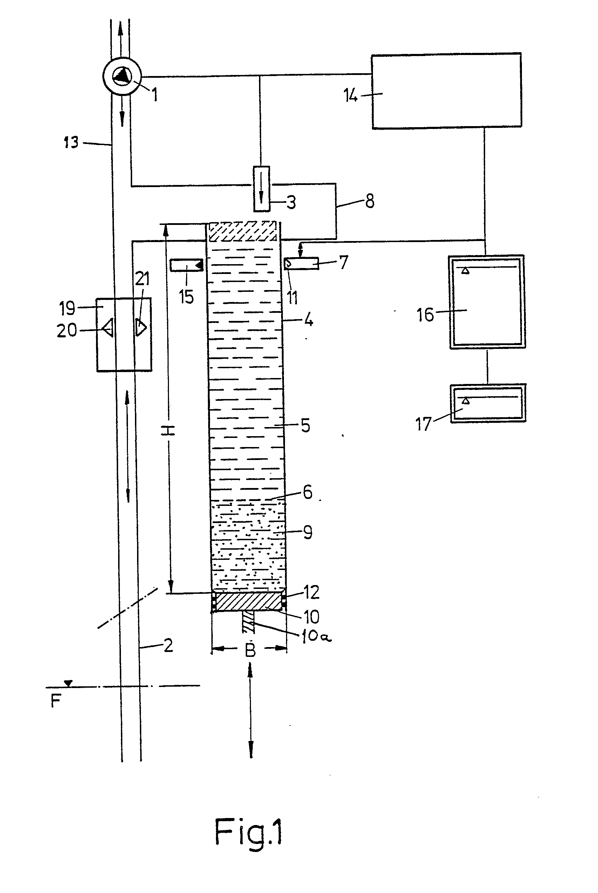

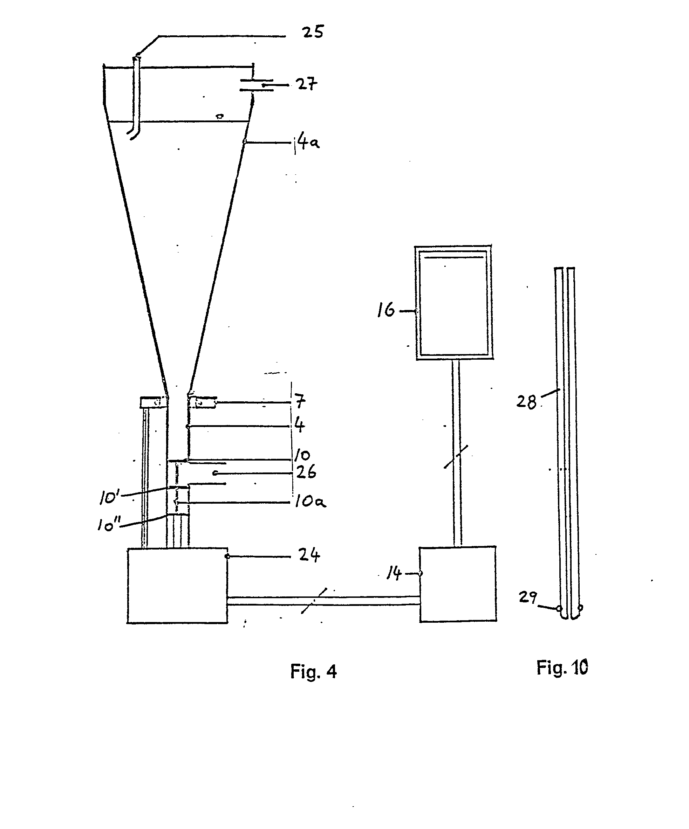

Während bei dem bisher beschriebenen Verfahren die Oberseite (Grenzschicht) 6 der Feststoffschicht 9 in ihrer Höhe dadurch gemessen wurde, daß der Abtastkopf 7 zu dieser Oberseite herunterfuhr, kann auch der Kolben 10 die gesamte Feststoffschicht 9 langsam nach oben drücken, bis die Oberseite 6 den Abtastkopf 7 erreicht. Der Weg des Kolbens 10 und der am Kolben befestigten Kolbenstange zeigt damit die Lage der Grenzschicht 6 an. Eine unterhalb des Kolbens angeordnete Antriebsvorrichtung 24 für die Kolbenstange 10a (Fig. 4) weist einen Drehpotentiometer auf, der mittelbar oder unmittelbar von der Kolbenstange 10a angetrieben wird und die Stellung des Kolbens 10 angibt. Durch ein exaktes Steuern des Auf- und Abfahrens und Anhaltens des Kolbens 10 an gewünschten Stellen ist es auch möglich, einen bestimmten Teil bzw. Prozentsatz der im Meßrohr 4 befindlichen Flüssigkeit 5 durch Hochfahren des Kolbens 10 aus dem Meßrohr 4 herauszudrücken und danach den Kolben 10 wieder in seine untere Lage zu fahren, so daß über der Flüssigkeit 5 im Meßrohr 4 ein der herausgedrückten Wassermenge entsprechender Luftraum entsteht. In diesen oberen freien Raum des Meßrohres 4 kann Verdünnungswasser durch eine Leitung eingebracht werden, die in den Fig. 1 bis 3 nicht dargestellt ist und in Fig. 4 mit 25 bezeichnet ist. Damit kann über die in Fig. 1 nicht dargestellte Antriebsvorrichtung 24 die Menge des zur Verdünnung benötigten Wassers exakt und leicht bestimmt werden.

Das Meßrohr 4 kann, wie in Fig. 4 dargestellt, nach unten hin über die in Fig. 1 gezeigte Stellung des Kolbens 10 (Einfüllstellung) verlängert sein und in dieser Meßrohrverlängerung befindet sich eine seitliche Auslauföffnung 26, die bei der Einfüllstellung des Kolbens 10 durch den Kolben vom übrigen Meßrohr getrennt ist und durch Herunterfahren des Kolbens 10 geöffnet wird. Unterhalb des Kolbens 10 können weitere Kolben 10' und 10'' auf der Kolbenstange 10a vorgesehen sein, um die Führung des Kolbens zu verbessern.

Das Meßrohr 4 weist in dem in den Fig. 4 bis 10 gezeigten Ausführungsbeispiel an der Oberseite eine konische Erweiterung auf, die in Form und Abmessungen einem bekannten Sedimentierglas entspricht. Hierdurch wird ein exaktes Messen und Arbeiten mit konischen Sedimentiergläsern möglich. In der Nähe des oberen Randes der konischen Erweiterung 4a befindet sich eine Öffnung 27 in der Wandung, die als Ablauf, Einlauf und/oder Überlauf arbeitet. In dem oberen Bereich der Erweiterung 4a befindet sich auch die schon erwähnte Leitung 25, deren Auslaßöffnung tangential zur Außenwandung dieser Erweiterung liegt, wodurch ein Vermischen von Verdünnungswasser mit dem Inhalt des Meßrohres verbessert wird. Ferner kann durch diese Leitung 25 beim leerem Meßrohr Reinigungswasser eingebracht werden, das durch die tangentiale Auslaßöffnung der Leitung 25 an der Wandung innenkreis- oder spiralförmig entlangläuft und damit schnell, gründlich und bei geringen Wassermengen die Innenwandung von Schmutzpartikeln reinigt.

Um für spätere Untersuchungen und Messungen die Feststoffschicht 9 von der darüber befindlichen Flüssigkeit 5 zu trennen, kann in das Meßrohr 4 von oben das in Fig. 10 gezeigte Rohr 28 eingefahren werden, das einen geringeren Außendurchmesser aufweist als der Innendurchmesser des Meßrohres 4. Im Bereich des unteren Endes weist das Rohr 28 außen einen Dichtring 29 auf, der an der Innenwandung des Meßrohres 4 dichtend anliegt. Wird dieses mit seiner Unterseite als Stopfen wirkende Rohr 28 in das Meßrohr 4 so weit eingeschoben, daß die Unterseite des Rohres 28 etwa auf der Oberseite 6 der Feststoffschicht 9 aufliegt, so wird die im Meßrohr 4 befindliche Flüssigkeit 5 durch das Innere des Rohres 28 nach oben verdrängt. Danach wird durch ein nicht gezeigtes Ventil oder durch einen Finger die obere Öffnung des Rohres 28 verschlossen und die Feststoffschicht 9 kann durch die Auslauföffnung 26 durch Herunterfahren des Kolbens 10 abgelassen werden. Während des Herunterfahrens des Kolbens 10 wird das Rohr 28 nachgeführt.

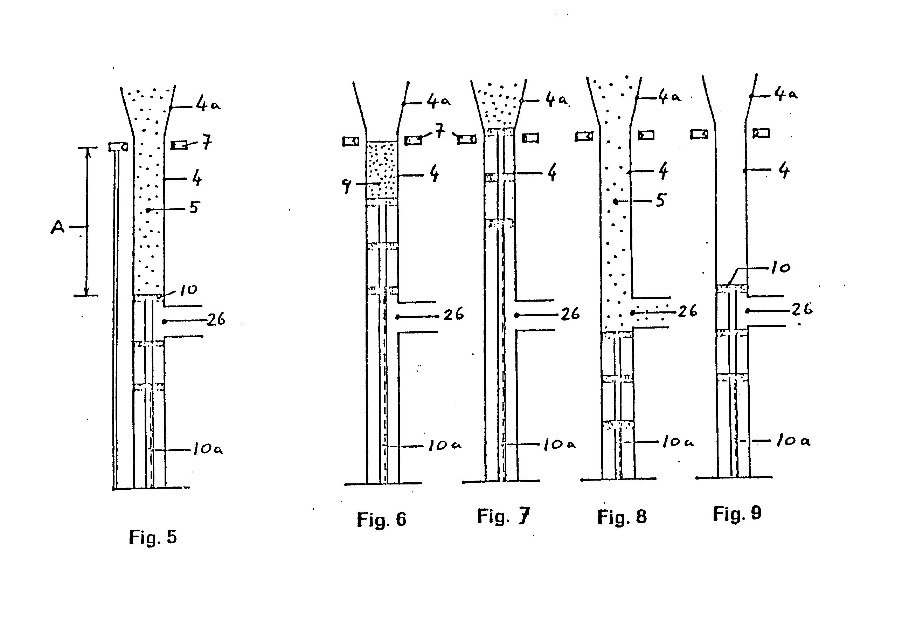

In den Fig. 5 bis 9 ist schematisch die Arbeitsweise der in der Fig. 4 gezeigten Vorrichtung dargestellt. Bei durch den Kolben 10 verschlossener Auslauföffnung 26 wird absetzbare Stoffe enthaltendes Wasser von oben in das Meßrohr 4 und in die konische Erweiterung 4a des Meßrohres eingefüllt. Nach einem bestimmten Zeitraum hat sich eine Feststoffschicht 9 gebildet. Die Höhe der Oberseite dieser Feststoffschicht wird dadurch gemessen, daß die Feststoffschicht 9 langsam bis an den Abtastkopf 7 herangefahren wird (Fig. 6), und der Weg des Kolbens 10 bzw. der Kolbenstange 10a gibt den Meßwert indirekt an, indem von dem Abstand A zwischen der Kolbenoberfläche und dem Abtastkopf 7 der Weg des Kolbens bzw. der Kolbenstange abgezogen werden muß.

Nach dem Messen der Höhe der Feststoffschicht 9 wird durch mehrmaliges Auf- und Abfahren des Kolbens 10 die Innenwandung des Meßrohres 4 gereinigt und danach der Kolben soweit abgefahren, daß die Auslauföffnung 26 offen ist (Fig. 8) und die Feststoffschicht 9 und die Flüssigkeit 5 ablaufen können. Danach wird durch die Leitung 25 sauberes Wasser oder eine Reinigungsflüssigkeit zur Reinigung der Innenwandung des Meßrohres 4 und der Erweiterung 4a eingegeben. Der Kolben 10 fährt darauf wieder in seine die Auslauföffnung 26 verschließende Ausgangslage (Fig. 9).

**Patentansprüche**

1. Vorrichtung zum automatischen Messen des absetzbaren Feststoffgehaltes eines Abwasser-Belebtschlamm-Gemisches, mit einem lichtdurchlässigen, senkrechten Meßrohr, das durch eine Pumpe mit der Flüssigkeit gefüllt wird, und mit einem fotoelektrischen Abtastkopf zum Auffinden der Lage der Grenzschicht der abgelagerten Feststoffschicht, dadurch gekennzeichnet, daß im Meßrohr (4) ein Kolben (10) angeordnet ist, durch den die im Meßrohr befindliche Flüssigkeit nach oben hin verdrängbar ist, wodurch die Grenzschicht (6) an den feststehenden Abtastkopf (7) bewegbar ist.

2. Vorrichtung nach Anspruch 1, dadurch gekennzeichnet, daß am Umfang des Kolbens (10) ringförmige Elemente (12) befestigt sind, durch die die Innenwand des Meßrohres (4) gereinigt wird.

3. Vorrichtung nach einem der Ansprüche 1 bis 2, dadurch gekennzeichnet, daß der Kolben (10) in der unteren Stellung den flüssigkeitsdichten Abschluß des Meßrohres (4) bildet.

4. Vorrichtung nach einem der Ansprüche 1 bis 3, dadurch gekennzeichnet, daß das Meßrohr (4) mit seiner oberen Öffnung in ein Speicher- oder Überlaufgefäß (8) mündet.

5. Vorrichtung nach Anspruch 4, dadurch gekennzeichnet, daß an der Oberseite des Überlaufgefäßes (8) eine Pumpe (1) angeschlossen ist, durch die die im Gefäß befindliche Luft ansaugbar ist und daß in die Unterseite des Gefäßes eine Flüssigkeitszufuhrleitung (2) mündet.

6. Vorrichtung nach Anspruch 5, dadurch gekennzeichnet, daß die Flüssigkeitszufuhrleitung (2) auch als Abflußleitung arbeitet.

7. Vorrichtung nach einem der Ansprüche 4 bis 6, dadurch gekennzeichnet, daß eine vom Flüssigkeitsstand im Überlaufgefäß (8) gesteuerte Schalteinrichtung (3) angeordnet ist, die die Pumpe (1) nach Erreichen einer bestimmten Füllhöhe abschaltet.

8. Vorrichtung nach einem der Ansprüche 1 bis 7, dadurch gekennzeichnet, daß der Kolben (10) zwischen der Ober- und Unterseite im Umfang eine ringförmige Nut (18) aufweist, in die eine Reinigungs- und/oder Gleitflüssigkeit einbringbar ist.

9. Vorrichtung nach einem der Ansprüche 1 bis 8, dadurch gekennzeichnet, daß an der Zuflußleitung und/oder der Zu- und Abflußleitung (2) eine Vorrichtung (19) angeordnet ist, die eine die Flüssigkeit durchdringende Schall- oder Strahlenquelle (20) zur Messung der Dichte des Feststoffes der Flüssigkeit aufweist.

10. Vorrichtung nach Anspruch 9, dadurch gekennzeichnet, daß ein Rechner vorgesehen ist, in den die Bewegung des Kolbens (10) über einen bestimmten Zeitabschnitt, die Stellung des Kolbens (10) am Ende der festgesetzten Meßzeit und die Dichte des Feststoffes in der in der Zuflußleitung (2) zeitweilig befindlichen Flüssigkeit eingebbar ist und der aus diesen drei Meßergebnissen mindestens einen Wert errechnet.

11. Vorrichtung nach einem der Ansprüche 1 bis 10, dadurch gekennzeichnet, daß das Meßrohr eine Ablauföffnung (26) an einer Stelle aufweist, die unterhalb des Meßrohrbereichs liegt, in der der Kolben (10) sich während des Füllens des Meßrohres (4) mit Flüssigkeit befindet.

12. Vorrichtung nach Anspruch 11, dadurch gekennzeichnet, daß das Meßrohr (4) unterhalb einer Ablauföffnung (26) eine Verlängerung aufweist, in die der Kolben (10) fahrbar ist.

13. Vorrichtung nach einem der Ansprüche 1 bis 12, dadurch gekennzeichnet, daß das Meßrohr (4) oberhalb des zylindrischen Meßbereichs sich nach oben hin konisch erweitert.

14. Vorrichtung nach Anspruch 13, dadurch gekennzeichnet, daß in der Nähe des oberen Randes der konischen Erweiterung (4a) eine Ablauföffnung (27) in der Wandung angeordnet ist.

15. Vorrichtung nach Anspruch 13 oder 14, dadurch gekennzeichnet, daß in der Nähe des oberen Randes der konischen Erweiterung (4a) eine tangentiale Einlauföffnung (25) für Verdünnungswasser und/oder Reinigungswasser angeordnet ist.

16. Vorrichtung nach einem der Ansprüche 13 bis 15, gekennzeichnet durch einen in den konischen Bereich bringbaren Stopfen (28), durch den der untere zylindrische Bereich des Meßrohres (4) von dem oberen konischen Bereich (4a) flüssigkeitsdicht trennbar ist.

17. Vorrichtung nach Anspruch 16, dadurch gekennzeichnet, daß der Stopfen (28) von einem unteren Bereich eines Rohres gebildet oder getragen wird.

18. Vorrichtung nach Anspruch 16 oder 17, dadurch gekennzeichnet, daß der Stopfen (28) an seiner Außenseite einen Abdichtring (29) aufweist.

19. Verfahren zum automatischen Messen des absetzbaren Feststoffgehaltes in einer Flüssigkeit mit einer Vorrichtung nach einem der Ansprüche 1 bis 18, dadurch gekennzeichnet, daß zuerst durch Absaugen der Luft im Überlaufgefäß (8) Flüssigkeit in das Überlaufgefäß eingesaugt wird und dabei vom Überlaufgefäß in das Meßrohr (4) läuft, bis dieses bei unten stehendem Kolben (10) gefüllt ist, daß die Flüssigkeit so lange weiter in das Überlaufgefäß (8) eingesaugt wird, bis eine vom Flüssigkeitsspiegel gesteuerte Schalteinrichtung (3) die Pumpe abschaltet, so daß die im Überlaufgefäß befindliche Flüssigkeit abläuft, daß nach einer Absetzzeit der Kolben (10) den im Meßrohr (4) befindlichen Inhalt (5, 9) soweit nach oben bewegt, bis die Grenzschicht (6) den Abtastkopf (7) erreicht, wobei der fotoelektrische Abtastkopf (7) die Höhe der Feststoffschicht (9) feststellt und angeschlossenen Geräten übermittelt, und daß darauf der Kolben (10) durch Hochfahren die im Meßrohr befindliche Flüssigkeit in das Überlaufgefäß verdrängt, aus dem sie abläuft.

**Revendications**

1. Dispositif pour la mesure automatique de la teneur en matière sédimentaire d'un mélange d'eaux résiduaires et des boues activés, avec un tube de mesure translucide et vertical, qui est rempli du liquide par une pompe, et avec une cellule photo-électrique à découvrir la position de la couche des matières sédimentaires, caractérisé en ce que dans le tube de mesure (4) un piston (10) est disposé par lequel le liquide se trouvant dans le tube de mesure est repoussable vers le haut, par où la couche (6) est mobile à cellule (7) fixée.

2. Dispositif selon la revendication 1, caractérisé en ce qu'au tour du piston (10) des éléments annulaires (12) sont fixés par lesquelles la paroi à l'intérieur du tube de mesure est nettoyée.

3. Dispositif selon une des revendications 1 à 2, caractérisé en ce que dans la position inférieure le piston (10) forme l'étanchéité du tube de mesure (4).

4. Dispositif selon une des revendications 1 à 3, caractérisé en ce qu'avec sa ouverture supérieure le tube de mesure (4) débouche dans un réservoir de stockage ou de trop-plein (8).

5. Dispositif selon la revendication 4, caractérisé en ce qu'à la face supérieure du réservoir de stockage (8) un pompe (1) est connectée, par laquelle l'air se trouvant dans le réservoir peut être aspiré et qu'une conduite d'approvisionnement de liquide (2) débouche dans la partie inférieure du réservoir.

6. Dispositif selon la revendication 5, caractérisé en ce que la conduite d'approvisionnement de liquide (2) fonctionne aussi comme une conduite d'écoulement.

7. Dispositif selon une des revendications 4 à 6, caractérisé en ce qu'un commutateur (3) commandé par le niveau du liquide dans le réservoir de trop-plein (8) est disposé, qui désacouple la pompe (1) après avoir atteint un certain niveau de remplissage.

8. Dispositif selon une des revendications 1 à 7, caractérisé qu'entre la partie supérieure et la partie inférieure au tour de piston, le piston (10) montre une rainure annulaire (18), dans laquelle un liquide de nettoyage et/ou de glissement peut être amené.

9. Dispositif selon une des revendications 1 à 8, caractérisé en ce qu'à la conduite d'approvisionnement et/ou à la conduite d'approvisionnement et d'écoulement (2) un dispositif (19) est ordonné qui présente une source sonore ou lumineux pénétrant le liquide pour la mesure de la densité de la matière sèche du liquide.

10. Dispositif selon la revendication 9, caractérisé en ce qu'un calculateur est prévu, dans lequel le mouvement du piston (10) pendant une période déterminée, la position du piston (10) à la fin du temps de mesure fixé et la densité de la matière sèche dans le liquide se trouvant temporairement dans la conduite d'approvisionnement (2) sont remettables et qui calcule de ces trois résultats de mesure au moins une valeur.

11. Dispositif selon une des revendications 1 à 10, caractérisé en ce que le tube de mesure présente une ouverture d'écoulement (26) à un endroit qui est situé au-dessous de la zone du tube de mesure, dans laquelle le piston (10) se trouve pendant le remplissage du tube de mesure (4).

12. Dispositif selon la revendication 11, caractérisé en ce que le tube de mesure (4) présente un prolongement au-dessous d'une ouverture d'écoulement (26), dans lequel le piston (10) peut s'en aller.

13. Dispositif selon une des revendications 1 à 12, caractérisé en ce qu'au-dessous de la zone de mesure cylindrique le tube de mesure (4) s'élargit d'une façon conique vers le haut.

14. Dispositif selon la revendication 13, caractérisé en ce que près du bord supérieur de l'élargissement conique (4a), une ouverture d'écoulement (27) est disposée dans la paroi.

15. Dispositif selon la revendication 13 ou 14, caractérisé en ce que près du bord supérieur de l'élargissement conique (4a) une ouverture d'approvisionnement (25) tangentielle est disposée pour de l'eau de dilution et/ou de l'eau de nettoyage.

16. Dispositif selon une des revendications 13 à 15, caractérisé par un bouchon (28) pouvant être apporté dans la zone conique, par lequel la zone cylindrique inférieure du tube de mesure (4) est séparable d'une façon étanche de la zone conique supérieure (4a).

17. Dispositif selon la revendication 16, caractérisé en ce que le bouchon (28) est formé de ou supporté par une partie inférieure d'un tuyau.

18. Dispositif selon la revendication 16 ou 17, caractérisé en ce que le bouchon (28) présente un joint annulaire à son côté extérieur.

19. Procédé pour la mesure automatique de la teneur en matière sédimentaire dans un liquide avec un dispositif selon une des revendications 1 à 18, caractérisé en ce qu'en premier lieu du liquide est aspiré dans le réservoir de trop-plein par l'aspiration de l'air dans le réservoir de trop-plein (8) et qu'en plus de cela le liquide coule du réservoir de trop-plein dans le tube de mesure (4) jusqu'à celui est rempli avec le piston (10) dans la position inférieure, que le liquide est aspiré dans le réservoir de trop-plein (8) aussi longtemps qu'un commutateur (3) commandé par le niveau du liquide se trouvant dans le réservoir de trop-plein s'écoule, qu'après un temps de sédimentation le piston (10) agite le contenu se trouvant dans le tube de mesure vers le haut aussi loin que la couche (6) atteint la cellule (7), à l'occasion de quoi la cellule photo-électrique (7) détermine l'hauteur de la couche de la matière sèche et transmet des instruments attachés, et qu'ensuite le piston (10) repousse le liquide se trouvant dans le tube de mesure par un mouvement vers le haut dans le réservoir de trop-plein, duquel il s'écoule.

**Claims**

1. Apparatus for automatically measuring the precipitatable solids content of a waste water-activated sludge mixture comprising a translucent perpendicular measuring tube which is filled with the liquid by a pump, and a photoelectric sensing head for detecting the position of the interface of the deposited layer of solid matter, characterised in that disposed in the measuring tube (4) is a piston by means of which the liquid in the measuring tube can be displaced upwardly whereby the interface (6) is movable at the stationary sensing head (7).

2. Apparatus according to claim 1 characterised in that secured to the periphery of the piston (10) are annular elements (12) for cleaning the inside wall surface of the measuring tube (4).

3. Apparatus according to one of claims 1 and 2 characterised in that in the lower position the piston (10) forms the liquid-tight closure for the measuring tube (4).

4. Apparatus according to one of claims 1 to 3 characterised in that the measuring tube (4) opens by means of its upper opening into a storage or overflow vessel (8).

5. Apparatus according to claim 4 characterised in that a pump (1) is connected to the top of the overflow vessel (8), for sucking in the air in the vessel, and that a liquid feed conduit (2) opens into the underside of the vessel.

6. Apparatus according to claim 5 characterised in that the liquid feed conduit (2) also operates as a discharge conduit.

7. Apparatus according to one of claims 4 to 6 characterised in that there is provided a switching means (3) which is controlled by the level of liquid in the overflow vessel (8) and which switches off the pump (1) after a given height of filling is reached.

8. Apparatus according to one of claims 1 to 7 characterised in that the piston (10) is provided with an annular groove (18) in its periphery between its top and its bottom, into which groove a cleaning and/or lubricating liquid can be introduced.

9. Apparatus according to one of claims 1 to 8 characterised in that arranged on the feed conduit and/or the feed and discharge conduit (2) is a device (19) which has a source (20) of sound or rays, for passing through the liquid, for the purposes of measuring the density of the solid matter in the liquid.

10. Apparatus according to claim 9 characterised in that there is provided a computer into which can be entered the movement of the piston (10) over a given period of time, the position of the piston (10) at the end of the set measuring time and the density of the solid matter in the liquid which is temporarily in the feed conduit (2), and which computes at least one value from said three measuring results.

11. Apparatus according to one of claims 1 to 10 characterised in that the measuring tube has a discharge opening (26) at a location which is below the region of the measuring tube in which the piston (10) is disposed during the operation of filling the measuring tube (4) with liquid.

12. Apparatus according to claim 11 characterised in that below a discharge opening (26) the measuring tube (4) has an extension portion into which the piston (10) can be moved.

13. Apparatus according to one of claims 1 to 12 characterised in that above the cylindrical measuring region, the measuring tube (4) flares outwardly conically in an upward direction.

14. Apparatus according to claim 13 characterised in that a discharge opening (27) is provided in the wall, in the vicinity of the upper edge of the conically flared portion (4a).

15. Apparatus according to claim 13 or claim 14 characterised in that a tangential intake opening (25) for diluting water and/or cleaning water is arranged in the vicinity of the upper edge of the conically flared portion (4a).

16. Apparatus according to one of claims 13 to 15 characterised by a stopper (28) which can be introduced into the conical region for liquid-tightly separating the lower cylindrical region of the measuring tube (4) from the upper conical region (4a).

17. Apparatus according to claim 16 characterised in that the stopper (28) is formed or carried by a lower portion of a tube.

18. Apparatus according to claim 16 or claim 17 characterised in that the stopper (28) has a sealing ring (29) on its outside.

19. A process for automatically measuring the precipitatable solids content in a liquid by an apparatus according to one of claims 1 to 18 characterised in that liquid is first sucked into the overflow vessel by sucking out the air in the overflow vessel (8) and flows from the overflow vessel into the measuring tube (4) until the measuring tube is filled, with the piston (10) at the bottom, that the liquid continues to be sucked into the overflow vessel (8) until a switching means (3) which is controlled by the level of liquid switches off the pump so that the liquid in the overflow vessel runs out, that after a precipitation time the piston (10) moves the content (5, 9) of the measuring tube (4) upwardly until the interface (6) reaches the sensing head (7), the photoelectric sensing head (7) detects the height of the layer (9) of solid matter and transmits same to connected devices, and that thereupon the piston (10) by moving upwardly displaces the liquid in the measuring tube into the overflow vessel from which it drains off.

Fig.1

Fig.2

Fig.3

Fig. 4

Fig. 10

Fig. 5

Fig. 6        Fig. 7        Fig. 8        Fig. 9

0 017 712